(19) Europäisches Patentamt
European Patent Office
Office européen des brevets

(11) **EP 3 413 042 A1**

(12) **EUROPEAN PATENT APPLICATION**
published in accordance with Art. 153(4) EPC

(43) Date of publication:
**12.12.2018 Bulletin 2018/50**

(21) Application number: **17747046.5**

(22) Date of filing: **06.02.2017**

(51) Int Cl.:
*G01N 29/46* (2006.01)    *G01N 29/34* (2006.01)
*G01N 29/36* (2006.01)    *G01N 29/24* (2006.01)
*A61B 8/08* (2006.01)    *G01N 33/483* (2006.01)

(86) International application number:
**PCT/ES2017/070065**

(87) International publication number:
**WO 2017/134327 (10.08.2017 Gazette 2017/32)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **04.02.2016 ES 201630123**

(71) Applicant: **Universidad de Granada
18071 Granada (ES)**

(72) Inventors:
• **RUS CALBORG, Guillermo
18071 Granada (ES)**
• **MELCHOR RODRÍGUEZ, Juan Manuel
18071 Granada (ES)**
• **MASSÓ GUIJARRO, Paloma
18071 Granada (ES)**

(74) Representative: **Hoffmann Eitle
Hoffmann Eitle S.L.U.
Paseo de la Castellana 140, 3a planta
Edificio LIMA
28046 Madrid (ES)**

(54) **METHOD FOR OBTAINING DATA RELATING TO THE ELASTICITY OF MATERIALS, USING TORSIONAL WAVES**

(57)    The invention relates to a method of operating mode which, using a device for emitting and receiving sonic and/or ultrasonic torsional waves, can be used to obtain data relating to the consistency of elasticity of qua-si-incompressible solid media, preferably quasi-fluids if biological tissues, based on the separation of non-linear parameters

Figure 1

EP 3 413 042 A1

**Description**

**FIELD OF THE ART**

The present invention is comprised in the field of material analysis, particularly in analysis methods using signal processing.

Specifically, the invention relates to methods which allow obtaining data relating to the elasticity of materials.

**STATE OF THE ART**

Torsional waves are a spatial distribution of transverse waves propagating along an axis in which particle movement occurs along a circumference the center of which is said axis, such that the amplitude of the movement in the generating plane is proportional to the distance from the axis within the diameter of the transducer.

These waves propagate through solid and semi-solid media, but not through perfect liquids, so measuring the speed of sound in media of this type can be very useful for studying their structural characteristics.

A transducer is a device capable of transforming or converting a specific type of input energy into another different type of output energy. These devices include, among others, electromechanical transducers which transform electrical energy into mechanical energy in the form of bidirectional displacements elastically coupled with stresses.

Ultrasonic transducers emit and receive sonic and/or ultrasonic waves, which allows, based on solid mechanics, identifying changes in tissue consistency which may indicate the presence of tumors, and quantifying mechanical or physical changes in the tissue can anticipate certain pathologies sooner than other diagnostic techniques can. Furthermore, the operating mode of the torsion sensor for obtaining non-linear parameters provides description and itemization in physical terms, rendering a value that is linked to the mode in which the fibers and the matrix in which they are embedded undergo deformation.

The problem with quasi-compressible materials (soft tissues and gels), the Poisson's ratio of which is about 0.5, lies in their bulk modulus and shear modulus being different. P- and S-type waves with different magnitudes propagate in these materials, and spurious P-waves that predominate and mask the S-waves are generated, not allowing commercial devices to read the S-waves which provide information about the shear modulus. If the operating mode is used as a diagnostic mode differentiating a healthy tissue from a diseased tissue at the level of tissue fibers and their support matrices, this mode has no ionizing effects which are indeed found in other diagnostic means such as X-rays.

The propagation of torsional waves is correlated with shear modulus by means of the elastic wave propagation equations, whereas the propagation of longitudinal waves is correlated with bulk modulus. In soft tissues, the non-linearity parameters vary by several orders of magnitude, so by using ultrasonic transducers based on non-linear torsional waves, a sensitivity that greater than that obtained with ultrasonic transducers based on P- and S-waves can be achieved.

Non-linear torsional wave generators were unknown up until the invention of the device, but the origin of devices for obtaining non-linear parameters based on P- and S-waves can be traced to a thesis published in 2009 by Muir entitled One-Sided Ultrasonic Determination of Third Order Elastic Constants using Angle-Beam Acoustoelasticity Measurements. The main limitation of this technique is that the P-wave transducers propagate at an angle so that, by means of mode conversion, an S-wave, the non-linear parameters of which are analyzed, is generated. It is extremely difficult to transfer this method to the field of tissues and quasi-fluids and almost impossible to extrapolate it to *in-vivo* assays. This has only been studied up until now in metals or homogenous materials.

Techniques such as those described in [Cristian Pantea, Curtis F Osterhoudt, and Dipen N Sinha. Determination of acoustical nonlinear parameter $\beta$ of water using the finite amplitude method. Ultrasonics, 53(5):1012-1019, 2013] or [Pham Chi Vinh and Jose Merodio. On acoustoelasticity and the elastic constants of soft biological tissues. Journal of Mechanics of Materials and Structures, 8(5): 359-367, 2013] are also known to be useful for obtaining parameters for measuring acoustic non-linearity in water by means of a mixture of waves and measuring non-linearity in tissue by means of dynamic acoustoelastic testing, DAET, in tissue, respectively. These techniques cannot be readily extrapolated as a diagnostic method due to experimental difficulty and due to the fact that the non-linear parameters have never been separated depending on their physical and biological origin before.

There is therefore a need for a method for obtaining data relating to the elasticity or consistency of materials which allows differentiating the volumetric part (linked to deformations due to tensile and compression forces) from the offset part (linked to deformations due to shear forces) in the studied sample.

**OBJECT OF THE INVENTION**

The present invention relates to a method or operating mode, hereinafter *"method of the invention"* which, using a device capable of emitting and receiving sonic and/or ultrasonic torsional waves, allows obtaining data relating to the consistency or elasticity of quasi-incompressible solid media (with Poisson's ratio close to 0.5), preferably biological tissues or quasi-fluids, based on the separation of non-linear parameters.

In particular, the method of the invention allows identifying changes in the consistency or elasticity of the analyzed sample depending on the behavior of the shear modulus thereof, by means of analyzing the torsional waves propagated through the material and received by the device of the invention.

The method of the invention uses a method for generating and measuring ultrasounds by means of the non-conventional use of shear waves and/or surface instead of longitudinal waves as they are several orders of magnitude more sensitive to variations in the microstructure of the relevant material which are closely linked to the non-linear parameters of the material.

The method of the invention improves the current technique, making it possible to differentiate the volumetric part (linked to deformations due to tensile and compression forces) from the offset part (linked to deformations due to shear forces) in the studied sample so as to allow determining its elasticity or consistency characteristics.

The use of non-linear sonic or ultrasonic waves as a physical magnitude has other fundamental advantages. Firstly, it is a mechanical wave that can be controlled, and is therefore more sensitive to mechanical properties than any other indirect measurement. Secondly, the wave is generated in a low-energy regimen which is more sensitive to variations in non-linear tissue parameters than waves generated at a high-energy regimen are.

Another object of the invention is a system, hereinafter "_system of the invention_," comprising the means required for carrying out the method of the invention.

Another object of the invention is a computer program comprising instructions for making a computer carry out the method of the invention. Likewise, other objects of the invention are a computer-readable storage medium comprising program instructions capable of making a computer carry out the method of the invention and a transmissible signal comprising program instructions capable of making a computer carry out the method of the invention.

By way of example, it can be obtained using the method of the invention parameters that are useful for identifying, based on solids mechanics, changes in non-linear parameters that define behavioral and state changes in materials manifested through changes in their elastic parameters which in turn control the propagation of waves going through them.

By means of analyzing these changes in elastic parameters, the presence and the type of tumors and any disorder manifesting as changes in said elastic parameters can be identified.

## DESCRIPTION OF THE DRAWINGS

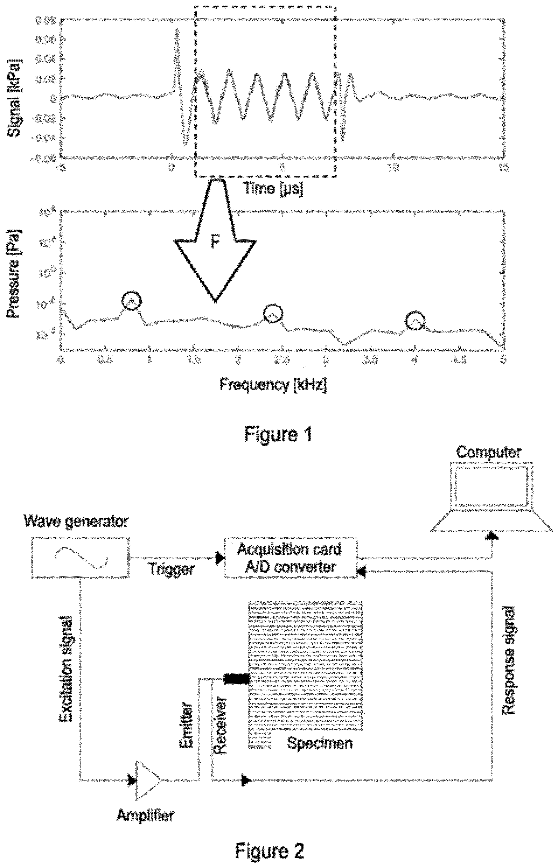

Figure 1 depicts the measurement made on a tissue surface, in this case on the operator's finger itself. The top part indicates with a dashed line the signal received in the time domain and the selected time window. The bottom part shows the frequency spectrum of the signal selected by means of Fourier transform, where the fundamental value (excitation frequency value) and harmonics value (multiples of the foregoing) are quantified.

Figure 2 depicts of a system which allows carrying out the method of the invention.

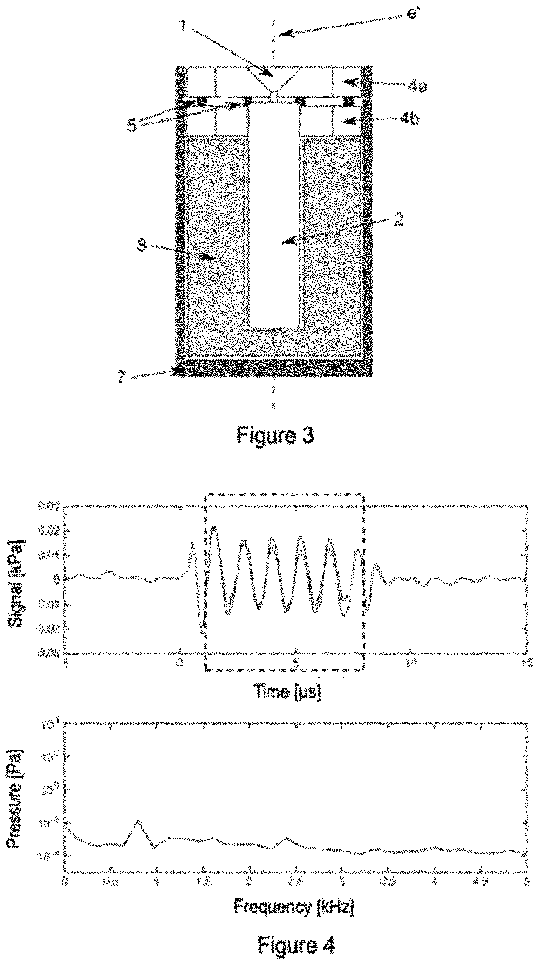

Figure 3 schematically depicts the torsional wave emitter and receiver device in which (1) represents the contact element, (2) the electromagnetic actuator, (4a) and (4b) the front and back rings, (5) the piezoelectric elements, (7) the casing containing all the elements, (8) the attenuator element, and (e') the axis of the receiver and the emitter.

Figure 4 depicts the measurement of a quasi-fluid-type material, in this case silicone. The obtained signal is located in the top part together with the selected time window, indicated with a dashed line. The bottom part of the graph shows the frequency spectrum of the fundamental and second harmonics which are essential for calculating the conventional acoustic non-linearity parameter.

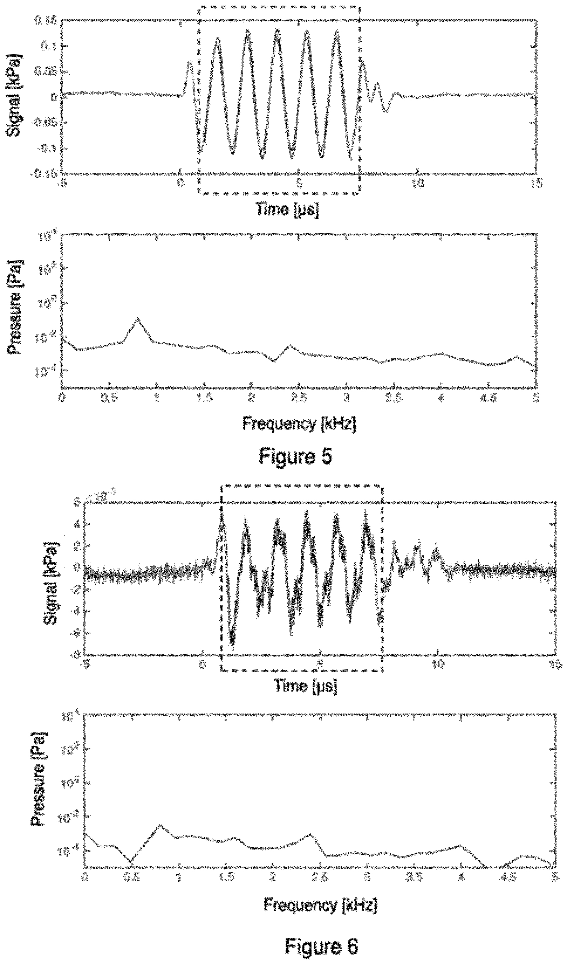

Figure 5 depicts the measurement of a tissue, in this case a connective tissue. The obtained signal and the selected time window, indicated with a dashed line, are located in the top part and the frequency spectrum of the fundamental and second harmonics which are essential for calculating the conventional acoustic non-linearity parameter are located in the bottom part of the graph.

Figure 6 depicts the measurement of a tissue, in this case soft chicken liver tissue, using an excitation energy of 10 V. The obtained signal and the selected time window, indicated with a dashed line, are located in the top part and the frequency spectrum of the fundamental and second harmonics which are essential for calculating the conventional acoustic non-linearity parameter are located in the bottom part of the graph.

**EXPLANATION OF THE INVENTION**

**Definitions:**

Throughout the present invention, *"wave train"* will be understood as a set of two or more identical sinusoidal wave cycles. In other words, two or more cycles having the same sinusoidal wave will be emitted in order to emit a wave train.

*"Selecting a time window"* of a wave determined by $\{(t, f(t)), t \geq 0\}$ defines the selection of an interval $[t_0, t_1]$ of the time domain of the wave whose wave image or function, $f([t_0, t_1])$, comprises one or more complete cycles.

Throughout the present description, *"specimen"* will be understood as material or sample of material, preferably a tissue, a tissue culture or a cell culture, through which waves emitted by the transducer are made to pass in order to learn about its structural characteristics (elastic parameters, viscoelastic parameters, microstructural geometry, porous, or energy dissipation models, among others). For purposes of the present document, an animal, preferably a human being, through which waves are made to pass, will also be considered a *"specimen."*

*"Electromechanical actuator"* will be understood as a device capable of transforming electrical energy into a movement, particularly a rotational movement. In a particular embodiment suitable for this invention, the electromechanical actuator is stimulated with an electrical signal generated by an electrical pulse generator and is capable of transforming said signal into a minimum fraction of a rotation which will be used to generate the wave that is subsequently analyzed. An example of actuators of this type may consist of an electromagnetic motor.

*"Electrical signal"* will be understood as an electrical magnitude the value of which depends on time. For purposes of the present invention, constant magnitudes will be considered as particular cases of electrical signals.

*Decimal notation*: This document uses the symbol "." as decimal point.

**Method of the invention**

The first object of the present invention is a method (*"method of the invention"*) for obtaining data relating to the elasticity of materials using torsional waves comprising the following steps of:

- emitting a torsional wave train on a specimen;
- selecting a time window of the received wave, originating from reflection on the specimen;
- calculating the Fourier transform of the wave function determined by the foregoing selection of time window;
- extracting the amplitudes from the fundamental harmonics, a, and from at least one of the harmonics of the second order, b, or higher;
- calculating one or more non-linearity parameters based on the extracted amplitudes of the harmonics.

By way of illustration, Figure 1 depicts the measurement made on the operator's finger. The top part shows the signal received, after the emission of a torsional wave train, in the time domain, converted to acoustic pressure units (kPa), and the selected time window, indicated with a dashed line, which describes a time domain corresponding to 5 cycles of the signal. The bottom part shows the frequency spectrum of the signal selected by means of Fourier transform, where the fundamental value (value for the excitation frequency) and harmonics value (multiple of the foregoing) are quantified in acoustic pressure units.

The non-linearity parameters, such as constitutive non-linearity $\beta$ or $\delta$, or conventional mechanical non-linearity parameters such as TOECs (third order elastic constants) or others, are parameters that are linked to the elasticity of materials and are used, for example, as a marker for the diagnosis of processes and pathologies in soft tissues.

The method of the invention uses non-linear torsional waves at several frequencies, the propagation speed of which depends directly on shear modulus, as the main indicator of soft tissue consistency. The use of torsional waves offers a higher sensitivity in the detection of irregularities in tissue consistency and has the advantage of eliminating almost all compression waves which contaminate the signal due to their complex propagation modes.

The main advantage of the method of the invention lies in the attainment of non-linearity parameters which allow characterizing the materials using constitutive and/or mechanical non-linearity parameters, the resolution of which is between three and six orders greater than the conventional linear parameters, offering a view at the microstructural level with respect to the onset of pathologies or changes of histological state in the case of tissues, or of microarchitecture in the case of inert materials, which translates into an early and accurate detection of potentially pathological relevant changes in the material.

Each of the features of the method of the invention and the different alternatives giving rise to particular embodiments of said method are described in more detail below:

Emitting a torsional wave train on a specimen

In a particular embodiment, the emitted wave train consists of between 2 and 80 cycles, preferably between 3 and 10 cycles.

Preferably, the excitation energy used for generating the wave train, in terms of the maximum amplitude of the preceding sinusoidal wave, is comprised between 0.1 V and 20 V, preferably between 2 and 10 V, and the sinusoidal excitation frequency is in the range between 100 Hz and 100 kHz, preferably between 500 Hz and 5 kHz.

It is not easy to obtain this excitation energy when generating torsional waves, so a torsional wave emitter device comprising an electromechanical actuator as will be described below is preferably used.

In a particular embodiment, the specimen is grounded such that the electromagnetic noise is minimized and the signal quality improves.

Selecting the time window

A time window of the received wave, originating from reflection on the specimen, must be selected such that it occupies a whole number of cycles to prevent the Fourier transform thereof from containing artifacts, where "artifact" is understood as those significant energies at frequencies different from the fundamental excitation frequency and its multiples or harmonics. Preferably, if $C$ is the total number of cycles having reflected wave, a time window consisting of the domain with a length associated with a natural number $c$, $c \leq C$, of wave cycles is selected, commencing at the moment of the transient fraction of the first cycle, particularly, halfway through the first cycle.

The determination of the moment in which the time window commences is in accordance with the heuristics determined by the type of material to be analyzed. Nevertheless, in another more particular embodiment, the time window commences at the moment of the transient fraction of the first cycle and is associated with a number of cycles comprised between $C - 2$ and $C$ where $C$ is the total number of cycles having reflected wave. In other words, the time window commences at the moment after the commencement of the received wave cycles and is associated with a number of cycles comprised between $C - 2$ and $C$, where $C$ is the total number of cycles having reflected wave, excluding significantly transient components.

Generally, the greater the selection of the time window is, i.e., the larger the number of cycles associated with selected domain is, the higher the resolution used for analyzing the recorded signal will be.

Calculating non-linearity parameters

By using the method of the invention, the behavior and the state of the tissue can be studied in terms of conventional constitutive non-linearity parameters (by analyzing the relationships existing between fundamental harmonics and harmonics of the order higher than or equal to two) such as $\beta$ or $\delta$, or conventional mechanical non-linearity parameters such as TOECs (third order elastic constants) or others.

In a particular embodiment, once the Fourier transform over the selected time window has been calculated and the amplitudes of harmonics extracted, the constitutive non-linearity or elasticity parameters can be calculated by means of the formula:

$$\circ \quad \beta_n = f\left(\frac{b}{a^n x^{n-1}}\right)$$

where $x$ is the shortest distance between the emitter and receiver, and $n$ is the order of the harmonics that is analyzed.

Repeating the method for minimizing noise

In another particular embodiment, the elasticity parameters of the specimen are obtained by repeating the method of the invention $r$ times, where $r \geq 2$, using identical wave trains with a time interval $T > 0$ between the emission of each wave train and calculating the mean of the calculated non-linearity parameters.

The time interval, $T$, is preferably greater than or equal to 5 times the duration of the emitted wave train.

Noise is significantly reduced with this average, whereas the time interval prevents the overheating of the emitter.

**Devices suitable for carrying out the method**

The emitted torsional waves must preferably have a high signal magnitude, preferably greater than 2 mV, more preferably greater than or equal to 5 mV, so the wave train will be emitted with a sonic and ultrasonic torsional wave emitter device comprising an electrical signal generator connected to an electromechanical actuator which is in turn attached to the contact element, such that when the actuator receives electrical signals, it induces rotational movement of the contact element and upon contacting the specimen, said contact element induces a torsional wave that goes through said specimen.

Any electronic circuit digitalizing the electrical signals at the desired frequencies can be used as the electrical signal generator. Another example of an electrical signal generator may be an oscilloscope, as it allows emitting an electrical signal with a variable voltage over a specific time.

In a preferred embodiment, the electrical signal used for stimulating the electromechanical actuator is an oscillating signal, more preferably a sine-wave signal, and even more preferably a sinusoidal signal, in the cycles claimed in "duty cycle" or work cycle between 1% and 20%, preferably 5%, to prevent overheating the device.

The electromechanical actuator comprising the emitter is preferably covered by a Faraday cage which eliminates electronic noise. Specifically, the electromechanical actuator is wrapped with a conductive covering acting as a Faraday cage.

## System of the invention

Another object of the invention is a system comprising the means required for carrying out the method of the invention.

Particularly, the system comprises means for emitting torsional waves, means for receiving torsional waves, and a processor suitable for executing instructions which allow carrying out the method of the invention.

More particularly, the system (Figure 2) comprises an emitter device which is connected, through an amplifier, to a wave generator controlled by a computer by means of an analog/digital converter, and a torsional wave receiver device sending the received signal to an analog/digital converter, which sends a digital signal to the computer that processes said signal according to the method of the invention.

In another particular embodiment, the system further comprises means which allow grounding the specimen. By way of example, these means can consist of a ground connection connected to the support on which the specimen is placed.

In a particular embodiment, the system includes a direct connection between the specimen and the ground connection of the transducer receiving channel. This connection will act as a reference for the input of the received signal.

## Implementing the method of the invention

A fourth object of the invention is a computer program comprising instructions making a computer, connected to the means making up the system of the invention, carry out the method of the invention.

The invention covers computer programs which can be in the form of a source code, an object code, or an intermediate code which is between a source code and an object code, such as in a partially compiled form, or in any other form suitable for use in the implementation of the processes according to the invention. In particular, the computer programs also cover cloud applications implementing the method of the invention.

These programs can be included in or on a medium suitable to be read, hereinafter, a "carrier medium" or a "carrier." The carrier medium can be any entity or device capable of holding the program. When the program is incorporated in a signal that can be transported directly through a cable or another device or means, the carrier medium may be formed by said cable or another device or means. As a variant, the carrier medium may be an integrated circuit in which the program is included, the integrated circuit being adapted to execute or to be used for executing the corresponding processes.

By way of example, the programs may be incorporated in a storage medium, such as a ROM memory, a CD ROM memory, or a semiconductor ROM memory, a USB memory, or a magnetic recording medium, for example, a floppy disk or a hard disk. Alternatively, the programs may be supported in a transmissible carrier signal. For example, said signal may be an electrical or optical signal that may be transported through an electric or optical cable, by radio, or through any other means.

In this sense, another object of the invention is a computer-readable storage medium comprising program instructions capable of making a computer carry out the method of the invention.

Finally, the last object of the invention relates to a transmissible signal comprising program instructions capable of making a computer carry out the method of the invention.

## EMBODIMENT

The embodiment of the method of the invention for obtaining data relating to the elasticity of different materials including tissues, among others, is proposed in a non-exclusive manner.

A system (Figure 2) consisting of a device for emitting and receiving torsional waves controlled by a computer that executes instructions for carrying out the method has been used for carrying out the method of the invention.

Specifically, the emitter is connected, through an amplifier, to a wave generator controlled by the computer by means of an analog/digital converter. The receiver in turn sends the received signal to the analog/digital converter and it is received by the computer which processes the received signal according to the method of the invention.

In turn, the device for emitting and receiving of torsional waves (Figure 3) comprises:

- a frustoconical-shaped contact element (1) made from PLA, the larger base of which comes into contact with the specimen and the smaller base of which is fixed to the shaft of the electromechanical actuator;
- an electromechanical actuator (2) consisting of a miniaturized motor 4 mm in diameter, fixed to the rear end (smaller base) of the contact element;
- an oscilloscope connected to the electromechanical actuator such that it transmits an electrical signal which the actuator transforms into rotational movement which the contact element converts into shear wave when it comes into contact with the specimen:

- an aluminum sheet arranged forming a covering for the electromechanical actuator and its conductive elements, and connected to the negative cable of the electromechanical actuator, such that it acts as a Faraday cage;
- A receiver formed by:

  ○ a first ring (4a) made from a plastic material, preferably PLA, having an outer diameter of 17 mm, an inner diameter of 13 mm, and a thickness of 5 mm;
  ○ a second ring (4b) made from a plastic material, preferably PLA, having an outer diameter of 17 mm, an inner diameter of 13 mm, and a thickness of 5 mm, placed parallel to the first ring;
  ○ a conductive covering located on the inner faces of each ring, such that it is in contact with the electrodes and works as an electrode;
  ○ 4 piezoelectric elements (5) made from piezoelectric ceramic PZT-4 or PZT-5, with dimensions of 1.5 x 1.5 x 2.5 mm, fixed to the rings. These piezoelectric elements are polarized in the circumferential direction parallel to the rings, whereas the electrodes are located in the attachment between the piezoelectric elements and the inner face of the rings, and

where the piezoelectric elements and wiring are attached to the electrodes with a conductive silver resin.

- a casing (7) adapted to the diagnostic device and made from PLA assuring the functionality of the device with its corresponding attenuating elements (8) and maintaining the relative arrangement between the emitter and the receiver such that their axes of rotation (e') coincide with one another and the front part of the contact element and the outer part of the front disk remain on the same plane.

For hygienic purposes, the transducer is coated with a latex membrane adapted to the shape of the device. The use of latex assures the dissipation of the wave travelling through it with an adapted involution between the emitter and the receiver. Using the preceding device, the elasticity of a silicone sample, a connective tissue sample, and a chicken liver tissue sample has been analyzed by executing, by means of instructions interpreted by the computer, the method of the invention with the following characteristics:

- A wave train with energies of 5 V and 10 V, and a frequency of 800 Hz, generated on the specimen is emitted through the emitter.
- The reflected wave is captured with the receiver and a time window of the received signal (Figures 3, 4, and 5) which includes the time fraction in which the wave form is cyclical, in the sense that the transient components are negligible, is selected.
- The Fourier transform is calculated for converting the function selected in the time window to the frequency domain.
- The amplitude of the second harmonics ($b$) and of the fundamental harmonics ($a$) is quantified, and constitutive non-linearity, $\beta$, is calculated by means of the formula $\beta = \dfrac{f\left(\dfrac{b}{a^n x^{n-1}}\right)}{}$, where x is the shortest distance between the emitter and receiver, in this case 2.3 mm, and $n$ is the order of the harmonics that is analyzed, in this case 2.

The method of the invention was carried out by emitting torsional waves with different energies on a silicone sample (Figure 4), a connective tissue sample (Figure 5), and a chicken liver sample (Figure 6).

The wave train used was a sinusoidal wave which was repeated for 6 cycles and emitted with an excitation energy of 5 and 10 V and a sinusoidal excitation frequency of 800 Hz.

The selection of the time window commenced halfway (50%) through the first cycle, and the domain associated with 5 cycles of the received wave (c=5) is selected.

The method was cyclically repeated for 50 times ($r$=50) with a time interval, $T$, of 80 milliseconds during which there was no excitation (0 V) in order to calculate the average of 50 measurements and to thereby reduce noise.

The results obtained after the test are shown in Table 1.

| Type of sample | Energy (V) | Frequency (Hz) | $\rho$ (kg/m$^3$) | Cs (m/s) | Z | $\mu$ (kPa) | $\beta^{\mathrm{T}}$ |
|---|---|---|---|---|---|---|---|
| Silicone | 5 | 800 | 1100 | 13.2 | 13200 | 0.16 | -400$\pm$3 |
| Connective tissue | 5 | 800 | 1000 | 90 | 90000 | 8.1 | -8000$\pm$0 |
| Liver tissue | 5 | 800 | 1000 | 4 | 4000 | 0.016 | -88$\pm$20 |
| Silicone | 10 | 800 | 1100 | 13.2 | 13200 | 0.16 | -400$\pm$3 |
| Connective tissue | 10 | 800 | 1000 | 90 | 90000 | 8.1 | -8000$\pm$0 |
| Liver tissue | 10 | 800 | 1000 | 4 | 4000 | 0.016 | -88$\pm$20 |

Table 1. Results obtained by applying the method on different samples. The energy of the emitted wave depends on the voltage (V) used to cause the excitation of the emitter, the frequency (Hz) is the frequency of the emitted wave, $\rho$ is the density of each of the materials, $C_s$ is the propagation speed of S-waves in each of the materials, Z is the coefficient of transmission of S-waves of each material, $\mu$ is the shear modulus of the different materials, and $\beta^{\mathrm{T}}$ is the conventional ultrasound coefficient of non-linearity of the first transverse order obtained after performing the method of the invention.

## Claims

1. Method for obtaining data relating to the elasticity of materials using torsional waves comprising the following steps of:

   - emitting a sonic or ultrasonic torsional wave train on a specimen;
   - selecting a time window of the received wave, originating from reflection on the specimen;
   - calculating the Fourier transform of the wave function determined by the foregoing selection of time window;
   - extracting the amplitudes from the fundamental harmonics, $a$, and from at least one of the harmonics of the second order, $b$, or higher;
   - calculating one or more non-linearity parameters based on the extracted amplitudes of the harmonics.

2. Method according to the preceding claim, **characterized in that** the emitted wave train consists of between 2 and 80 cycles, preferably between 3 and 10 cycles.

3. Method according to any of the preceding claims, **characterized in that** the emitted torsional waves have a signal magnitude greater than 2 mV, more preferably greater than or equal to 5 mV.

4. Method according to any of the preceding claims, **characterized in that** the excitation energy used for generating the wave train, in terms of the maximum amplitude of the preceding sinusoidal wave, is comprised between 0.1 V and 20 V, preferably between 2 and 10 V.

5. Method according to any of the preceding claims, **characterized in that** the sinusoidal excitation frequency is in the range between 100 Hz and 100 kHz, preferably between 500 Hz and 5 kHz.

6. Method according to any of the preceding claims, **characterized in that** the time window commences at the moment after the commencement of the received wave cycles and is associated with a number of cycles comprised between $C$ - 2 and $C$, where $C$ is the total number of cycles having the wave reflected, excluding significantly transient components.

7. Method according to any of the preceding claims, **characterized in that** once the Fourier transform over the selected time window has been calculated and the amplitudes of the fundamental harmonics, *a*, and of at least one of the harmonics of the second order, *b*, or higher, have been extracted, the constitutive non-linearity or elasticity parameters are calculated by means of the formula:

$$\circ \quad \beta_n = f\left(\frac{b}{a^n x^{n-1}}\right)$$

where x is the shortest distance between the emitter and receiver, and *n* is the order of the harmonics that is analyzed.

8. Method for obtaining the elasticity parameters of a specimen which repeats the method according to any of the preceding claims at least twice by using identical wave trains with a time interval, *T* > 0, between the emission of each wave train, and calculating the mean of the calculated non-linearity parameters.

9. Method according to preceding claim, **characterized in that** the time interval, *T*, is greater than or equal to 5 times the duration of the emitted wave train.

10. Method according to any of the preceding claims, **characterized in that** the wave train is emitted with a sonic and/or ultrasonic torsional wave emitter device comprising an electrical signal generator connected to an electromechanical actuator which is in turn attached to the contact element, such that when the actuator receives electrical signals, it induces rotational movement of the contact element and upon contacting the specimen, said contact element induces a torsional wave that goes through said specimen.

11. Method according to the preceding claim, **characterized in that** the electrical signal used for stimulating the electromechanical actuator is a signal in the cycles claimed in "duty cycle" or work cycle between 1% and 20%, preferably 5%.

12. System for obtaining data relating to the elasticity of materials using torsional waves, comprising means for emitting torsional waves, means for receiving torsional waves, and a processor suitable for executing instructions that allow carrying out the method according to any of the preceding claims.

13. System according to the preceding claim, comprising an emitter device which is connected, through an amplifier, to a wave generator controlled by a computer by means of an analog/digital converter, and a torsional wave receiver device sending the received signal to an analog/digital converter, which sends a digital signal to the computer that processes said signal according to the method according to any of claims 1 to 11.

14. Computer program comprising instructions to make a computer carry out the method according to any of claims 1 to 11.

15. Computer-readable storage medium comprising program instructions capable of making a computer carry out the method according to any of claims 1 to 11.

16. Transmissible signal comprising program instructions capable of making a computer carry out the method according to any of claims 1 to 11.

Figure 1

Figure 2

Figure 3

Figure 4

Figure 5

Figure 6

# INTERNATIONAL SEARCH REPORT

| | International application No. |
| --- | --- |
| | PCT/ES2017/070065 |

## A. CLASSIFICATION OF SUBJECT MATTER

**See extra sheet**

According to International Patent Classification (IPC) or to both national classification and IPC

## B. FIELDS SEARCHED

Minimum documentation searched (classification system followed by classification symbols)
G01N29, A61B8

Documentation searched other than minimum documentation to the extent that such documents are included in the fields searched

Electronic data base consulted during the international search (name of data base and, where practicable, search terms used)

INVENES, EPODOC, WPI, XPESP

## C. DOCUMENTS CONSIDERED TO BE RELEVANT

| Category* | Citation of document, with indication, where appropriate, of the relevant passages | Relevant to claim No. |
| --- | --- | --- |
| A | WO 2010012092 A1 (CT HOSPITALIER DE L UNIVERSITE et al.) 04/02/2010, paragraphs [2,10,11,41-45,57,60-71] | 1-16 |
| A | (HADJ HENNI ANIS et al.) "Shear wave induced resonance elastography of spherical masses with polarized torsional waves". Applied Physics Letters, VOL: 100 No: 13, Pags: 133702 – 133705. 26/03/2012 ISSN 0003-6951 Doi: 10.1063/1.3696300 | 1-16 |
| A | (SCHMITT et al.) "Ultrasound Dynamic Micro-Elastography Applied to the Viscoelastic Characterization of Soft Tissues and Arterial Walls". Ultrasound in Medicine and Biology, VOL: 36 No: 9, Pags: 1492 – 1503, 01/09/2010 ISSN 0301-5629 | 1-16 |

☒ Further documents are listed in the continuation of Box C.          ☒ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance. | | |
| "E" | earlier document but published on or after the international filing date | | |
| "L" | document which may throw doubts on priority claim(s) or which is cited to establish the publication date of another citation or other special reason (as specified) | "X" | document of particular relevance; the claimed invention cannot be considered novel or cannot be considered to involve an inventive step when the document is taken alone |
| "O" | document referring to an oral disclosure use, exhibition, or other means. | "Y" | document of particular relevance; the claimed invention cannot be considered to involve an inventive step when the document is combined with one or more other documents , such combination being obvious to a person skilled in the art |
| "P" | document published prior to the international filing date but later than the priority date claimed | | |
| | | "&" | document member of the same patent family |

| Date of the actual completion of the international search | Date of mailing of the international search report |
| --- | --- |
| 10/05/2017 | **(12/05/2017)** |

| Name and mailing address of the ISA/ | Authorized officer |
| --- | --- |
| OFICINA ESPAÑOLA DE PATENTES Y MARCAS Paseo de la Castellana, 75 - 28071 Madrid (España) Facsimile No.: 91 349 53 04 | F. Olalde Sánchez Telephone No. 91 3498469 |

Form PCT/ISA/210 (second sheet) (January 2015)

## INTERNATIONAL SEARCH REPORT

| International application No. |
| --- |
| PCT/ES2017/070065 |

### C (continuation). DOCUMENTS CONSIDERED TO BE RELEVANT

| Category * | Citation of documents, with indication, where appropriate, of the relevant passages | Relevant to claim No. |
| --- | --- | --- |
| A | (OUARED A et al.) "Frequency adaptation for enhanced radiation force amplitude in dynamic elastography". IEEE Transactions on Ultrasonics, Ferroelectrics and Frequency Control, VOL: 62 No: 8 Pags: 1453 – 1466, 08/2015 ISSN 0885-3010 (print) Doi: 10.1109/TUFFC.2015.007023 | 1-16 |
| A | (DOHERTY et al.) "Acoustic radiation force elasticity imaging in diagnostic ultrasound". IEEE Transactions on Ultrasonics, Ferroelectrics and Frequency Control, VOL: 60 No: 4 Pags: 685 – 701, 01/04/2013 ISSN 0885-3010 Doi: 10.1109/TUFFC.2013.2617 | 1-16 |
| A | (SARVAZYAN et al.) "Shear wave elasticity imaging: a new ultrasonic technology of medical diagnostics". Ultrasound in Medicine and Biology, VOL: 24 No: 9, Pags: 1419 – 1435, 01/12/1998 ISSN 0301-5629 Doi: 10.1016/S0301-5629(98)00110-0 | 1-16 |
| A | (MELCHOR et al.) "Torsional ultrasonic transducer computational design optimization". Ultrasonics, VOL: 54 No: 7 Pags: 1950 – 1962, 15/05/2014 ISSN 0041-624X Doi: 10.1016/j.ultras.2014.05.001 | 1-16 |

Form PCT/ISA/210 (continuation of second sheet) (January 2015)

<table>
<tr><td colspan="2" align="center"><b>INTERNATIONAL SEARCH REPORT</b><br>Information on patent family members</td><td colspan="2">International application No.<br><br>PCT/ES2017/070065</td></tr>
<tr><td align="center">Patent document cited<br>in the search report</td><td align="center">Publication<br>date</td><td align="center">Patent family<br>member(s)</td><td align="center">Publication<br>date</td></tr>
<tr><td align="center">WO2010012092 A1</td><td align="center">04.02.2010</td><td align="center">US2011130660 A1<br>CA2732334 A1<br>EP2310829 A1<br>EP2310829 A4</td><td align="center">02.06.2011<br>04.02.2010<br>20.04.2011<br>07.08.2013</td></tr>
</table>

Form PCT/ISA/210 (patent family annex) (January 2015)

## INTERNATIONAL SEARCH REPORT

International application No.

PCT/ES2017/070065

### CLASSIFICATION OF SUBJECT MATTER

*G01N29/46* (2006.01)
*G01N29/34* (2006.01)
*G01N29/36* (2006.01)
*G01N29/24* (2006.01)
*A61B8/08* (2006.01)
*G01N33/483* (2006.01)

Form PCT/ISA/210 (extra sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **MUIR.** One-Sided Ultrasonic Determination of Third Order Elastic Constants using Angle-Beam Acoustoelasticity Measurements. *thesis,* 2009
- **CRISTIAN PANTEA ; CURTIS F OSTERHOUDT ; DIPEN N SINHA.** Determination of acoustical nonlinear parameter β of water using the finite amplitude method. *Ultrasonics,* 2013, vol. 53 (5), 1012-1019

- **PHAM CHI VINH ; JOSE MERODIO.** On acoustoelasticity and the elastic constants of soft biological tissues. *Journal of Mechanics of Materials and Structures,* 2013, vol. 8 (5), 359-367